# EUROPEAN PATENT APPLICATION

(11) **EP 4 775 963 A1**
(43) Date of publication of application: **15.07.2026**
(21) Application number: 25151513.6
(22) Date of filing: 13.01.2025
(51) Int. Cl.: G01N 21/63, G01J 3/42

(54) **SPECTROSCOPIC MEASURING APPARATUS AND METHOD FOR MEASURING A SPECTRAL RESPONSE OF A SAMPLE, EMPLOYING AN AMPLIFICATION OF A FREE-INDUCTION DECAY LIGHT FIELD**

(71) Applicant: Max-Planck-Gesellschaft zur Förderung der Wissenschaften e.V., 80539 München (DE); Ludwig-Maximilians-Universität München, in Vertretung des Freistaates Bayern, 80539 München (DE); Center for Molecular Fingerprinting Research Nonprofit Limited Liability Company, 1093 Budapest (HU)
(72) Inventor: KRAUSZ, Ferenc, 85748 Garching (DE); KARPOWICZ, Nicholas, 80939 München (DE); MAK, Kafai, 80801 München (DE); WEIGEL, Alexander, 81245 München (DE)
(74) Representative: v. Bezold & Partner Patentanwälte - PartG mbB

(57) **Abstract**

A spectroscopic measuring apparatus 100 for measuring a spectral response of a sample 1 comprises a probe light source device 11 irradiating the sample with a probe light pulse 2 with a probe pulse duration, wherein an interaction of the probe light pulse 2 with the sample 1 results in a free induction decay light field 3 with a free induction decay duration greater than the probe pulse duration; an amplifier device amplifying the free induction decay light field 3; and a detector device 30 providing the spectral response of the sample by temporally resolved detecting the free induction decay light field 3, wherein the amplifier device is an optical parametric amplification (OPA) device 20 with a pump light source device 21 repeatedly creating a pump light pulse 5 having a pump pulse duration shorter than the free induction decay duration, and further including an optically non-linear medium 22, and the OPA device 20 is arranged for an optical parametric amplification of the free induction decay light field 3 by scanning the free induction decay light field 3 with the pump light pulse 5 repeatedly provided with a varying amplification delay relative to the free induction decay light field 3, and the detector device 30 includes a sampling light source device 31 repeatedly creating a sampling light pulse 4 with a sampling pulse duration shorter than the free induction decay duration, wherein the detector device 30 is arranged for time domain sampling the free induction decay light field 3 by scanning the free induction decay light field 3 with the sampling light pulse 4 repeatedly created with a varying sampling delay relative to the free induction decay light field 3, said sampling light pulse being provided synchronously with the pump light pulse 5. Furthermore, a spectroscopic measuring method for measuring a spectral response of a sample is described.

## Description

### Field of the invention

The invention relates to a spectroscopic measuring apparatus and to a spectroscopic measuring method for measuring a spectral response of a sample, in particular a biological sample, by field-resolved spectroscopy. Applications of the invention are available e. g., in the fields of biochemistry, medicine or material sciences.

### Prior art

In the present specification, reference is made to the following prior art illustrating the technical background of the invention:
[1] I. Pupeza et al. "Field-resolved infrared spectroscopy of biological systems" in "Nature" 577, 7788, 52-59 (2020);
[2] EP 3 037 805 B1;
[3] WO 2022/248894 A1; and
[4] Liu et al. "Optical-parametric-amplification-enhanced background-free spectroscopy" in "Optics Letters" 49, 11, 2914-2917 (2024)

It is generally known that broadband mid-infrared (MIR) spectroscopy (light frequencies e.g., in a range from 400 cm⁻¹ to 3300 cm⁻¹ (covering at least a half of this range), or light wavelengths e.g., in a range from 3 µm to 25 µm (covering at least a half of this range)) is useful for sensitive molecular sensing, e.g., biomedical sensing. Fourier transform infrared (FTIR) spectroscopy [1, 2] is an established standard tool for molecular sensing. A recently proposed alternative approach of broadband MIR spectroscopy is provided by field-resolved spectroscopy (FRS), wherein a few-optical-cycle, broadband MIR pulse excites the sample, and the full electric field including the molecular response in the wake of the pulse (free induction decay light field) is directly captured in a time-domain electro-optic sampling (EOS) measurement. Using FRS based on femtosecond MIR laser pulses, one can achieve higher dynamic range, sensitivity and specificity for molecular detection when compared to current state-of-the-art FTIR spectroscopy [1, 2].

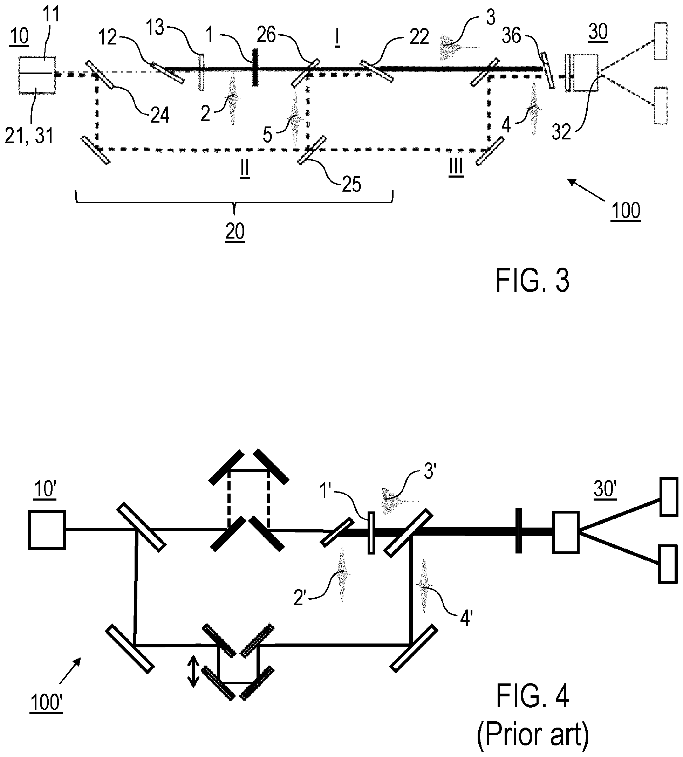

The FRS apparatus 100' of [2], as schematically illustrated in Figure 4 (prior art), comprises a probe light source 10' for providing spectrally broadened probe light pulses 2' (excitation pulses). An interaction of the probe light pulses 2' with the sample 1' results in the free induction decay light field 3' having a free induction decay pulse duration, which is greater than the probe pulse duration. The FRS apparatus 100' further comprises a detector device 30' for sensing the spectral response of the sample 1' by a temporally resolved detection of the free induction decay light field 3' by an EOS measurement. Electro-optical sampling of the free induction decay light field 3' is executed with sampling light pulses 4' created with the probe light source 10' as well.

Measuring the free-induction decay light field 3' is particularly advantageous, as this signal will not be masked by potentially large power fluctuation of the excitation pulse. However, the free-induction decay signal can be quite weak, and it decays rapidly after the excitation pulse, resulting in limitations in detecting substances at low concentrations. The low signal strength of the free-induction decay could in principle be circumvented by using a stronger excitation pulse, but doing so results in a risk of destroying the sample to be measured.

Thus, it has been suggested to amplify the free-induction decay light field. One method that has been proposed is to amplify the free-induction decay and the probe light pulse, with settings preferentially amplifying the former, using quantum cascade lasers QCL [3]. Ideally, only the free-induction decay light field, but not the probe light pulse, is amplified, with amplification switched on only after the probe light pulse has passed. However, in the QCL case, to initiate the optical amplification, electronics such as a photodiode and electronic amplifier will be required, which have rise-time and jitter on the order of >1 picosecond. These limit the ability to selectively amplify the free-induction decay close to but separate from the probe light pulse, which typically has durations in the tens of femtoseconds.

Optical parametric amplification represents another way to boost the free-induction decay signal, as recently proposed in [4]. With no electronics required in the delay mechanism, the rise time and the timing jitter can be significantly reduced to the femtosecond timescale. However, the OPA scheme of [4] requires a pump pulse for the OPA that covers a significant portion of the free-induction decay duration, and therefore requires a relatively long pulse (tens of picosecond as stated in [4]) so that the free induction decay light field cannot be sampled with time-resolution and a portion of the probe light pulse may be amplified together with the free-induction decay field.

Furthermore, for the relatively long pulses employed in [4], to achieve the required peak power that still amplifies the free-induction decay, the OPA pump pulse needs to contain significant pulse energies. These are difficult to provide from laser systems with MHz repetition rates and will need to be generated via kHz repetition-rate laser systems. On the other hand, it is beneficial to use excitation pulses at MHz repetition rate to induce the free-induction decay in the sample in the first place (before the OPA stage), because doing so can increase the signal-to-noise level without resorting to sample-damaging peak power of the excitation pulse.

### Objective of the invention

The objective of the invention is to provide an improved spectroscopic measuring apparatus and method for measuring a spectral response of a sample by means of field-resolved spectroscopy, being capable of avoiding limitations of conventional techniques. In particular, the objective of the invention is to measure the spectral response with increased sensitivity and/or to allow detecting substances at low concentrations, and/or with the capability of employing high sampling rates, e.g., toward MHZ, and/or with the capability of time resolved measurements with FRS, in particular with a time resolution down to tens of femtoseconds, and/or with improved amplification of the free induction decay light field.

### Brief summary of the invention

This objective is solved by a spectroscopic measuring apparatus and spectroscopic measuring method, comprising the features of the independent claims, respectively. Preferred embodiments and applications of the invention arise from the dependent claims.

According to a first general aspect of the invention, the above objective is solved by a spectroscopic measuring apparatus being configured for measuring a spectral response of a sample, in particular a biological sample. The spectroscopic measuring apparatus comprises a probe light source device being arranged for an irradiation of the sample with a probe light pulse (excitation pulse) having a probe pulse duration, wherein an interaction of the probe light pulse with the sample results in a free induction decay light field having a free induction decay duration, which is greater than the probe pulse duration. Preferably, the probe light source device may comprise a fs laser source being capable of creating initial fs pulses e.g., with a duration in a range from 3 fs to 500 fs. The fs laser source may be combined with a difference frequency generation device being arranged for providing the probe light pulse with a MIR spectrum by an optical non-linear difference frequency generation driven by the initial fs pulses.

Furthermore, the spectroscopic measuring apparatus comprises an amplifier device being arranged for amplifying the free induction decay light field and a detector device being arranged for providing the spectral response of the sample by a temporally resolved detection of the amplified free induction decay light field.

According to the invention, the amplifier device is an optical parametric amplification (OPA) device including a pump light source device being arranged for repeatedly creating a pump light pulse having a pump pulse duration, which is shorter than the free induction decay duration, and further including an optically non-linear medium. The optically non-linear medium is arranged for receiving the free induction decay light field and the pump light pulse with a given temporal relationship and for an optical non-linear interaction of the free induction decay light field and the pump light pulse in an amplification time window provided by a current temporal overlap of the pump light pulse with the free induction decay light field. In particular, the current temporal overlap is determined by the temporal relationship of the free induction decay light field and the pump light pulse. The optical non-linear interaction is limited to the amplification time window. The free induction decay light field and the pump light pulse may propagate either collinearly or non-collinearly through the optically non-linear medium.

Furthermore, according to the invention, the amplifier device, in particular the optically non-linear medium thereof, is arranged for an optical parametric amplification of the free induction decay light field by scanning the free induction decay light field with the pump light pulse repeatedly provided with a varying amplification delay relative to the free induction decay light field. The temporal relationship of the free induction decay light field and the pump light pulse, in particular the time delay of the shorter pump light pulse relative to the longer free induction decay light field is called amplification delay. The free induction decay light field is scanned by step-wise changing the amplification delay, i.e., step-wise shifting the amplification time window along the free induction decay light field, and correspondingly step-wise amplifying sections of the free induction decay light field covered by the current temporal position of the pump light pulse.

Furthermore, according to the invention, the detector device includes a sampling light source device being arranged for repeatedly creating a sampling light pulse (or: gating light pulse) having a sampling pulse duration, which is shorter than the free induction decay duration, wherein the detector device is arranged for time domain sampling of the free induction decay light field by scanning the amplified free induction decay light field with the sampling pulse repeatedly created with a varying sampling delay relative to the free induction decay light field, said sampling light pulse being provided synchronously (temporally controlled in synchronism) with the pump light pulse. The temporal relationship of the free induction decay light field and the sampling light pulse, in particular the time delay of the shorter sampling light pulse relative to the longer free induction decay light field is called sampling delay. The free induction decay light field is scanned by step-wise changing the sampling delay, i.e., step-wise shifting a sampling time window along the free induction decay light field, and correspondingly step-wise sensing sections of the free induction decay light field covered by the current temporal position of the sampling light pulse. In particular, the duration of the sampling time window is determined by the sampling pulse duration. Preferably, the sampling pulse duration may be matched to the pump pulse duration. Particularly preferred, the sampling pulse duration may be equal to or shorter than the pump pulse duration.

According to a second general aspect of the invention, the above objective is solved by a spectroscopic measuring method for measuring a spectral response of a sample, in particular a biological sample. The spectroscopic measuring method comprises the steps of irradiation of the sample with a probe light pulse created with a probe light source device and having a probe pulse duration, wherein an interaction of the probe light pulse with the sample results in a free induction decay light field having a free induction decay duration, which is greater than the probe pulse duration, amplifying the free induction decay light field with an amplifier device, and providing the spectral response of the sample by a temporally resolved detection of the free induction decay light field with a detector device.

According to the invention, the amplifier device is an optical parametric amplification (OPA) device including a pump light source device repeatedly creating a pump light pulse having a pump pulse duration, which is shorter than the free induction decay duration, and further including an optically non-linear medium, and the amplifier device provides an optical parametric amplification of the free induction decay light field by scanning the free induction decay light field with the pump light pulse repeatedly provided with a varying amplification delay relative to the free induction decay light field. The detector device includes a sampling light source device repeatedly creating a sampling light pulse having a sampling pulse duration, which is shorter than the free induction decay duration, wherein the detector device provides a time domain sampling of the free induction decay light field by scanning the amplified free induction decay light field with the sampling pulse repeatedly created with a varying sampling delay relative to the free induction decay light field, said sampling pulse being synchronized with the pump light pulse. Preferably, the spectroscopic measuring method or an embodiment thereof is executed with the spectroscopic measuring apparatus according to the first general aspect of the invention or an embodiment thereof.

The free induction decay light field is a physical radiation response of the sample material and not under direct external control. It is a gradually decaying light field rather than a pulse in a physical sense. The decay starts immediately after the passing of the probe pulse, but can last e.g., picoseconds. Since there are multiple molecular oscillators of different frequencies in the sample oscillating together, they may create beat structures in the free induction decay light field that modulate the strength of the combined free induction decay light field signal.

The free induction decay duration may be defined with reference to an interval of the free induction decay light field starting just after the excitation with the probe light pulse, e.g. a time range where the amplitude of the free induction decay light field falls to a factor of 1/2 or 1/e. Depending on the sample to be investigated, the free induction decay duration may be defined larger or smaller than with these examples.

The sample to be investigated may comprise a solid, liquid and/or gaseous sample. Solid or liquid samples typically have a relative fast decay of the free induction decay light field up to the ps-range, while gaseous sample have a relative slow decay of the free induction decay light field up to the ns-range. Preferably, sample may comprise a biological sample, e. g., a liquid including a biological material, like biological particles, biological cells, cell groups, cell components and/or molecules, and/or a vapour including the biological material. Generally, the spectral response of the sample of interest is provided directly by the sensed free induction decay light field or by spectral data, like an amplitude spectrum, obtained from the sensed free induction decay light field as known from conventional FRS.

Advantageously and in contrast to [4], the optical parametric amplification is provided with time resolution, because the pump light source device creates the pump light pulse with a duration shorter than the free induction decay light field and the free induction decay light field is scanned with the pump light pulse. Successively, different temporal sections of the free induction decay light field are amplified, preferably covering the whole time range of interest of the free induction decay light field, e.g. a time range where the free induction decay light field falls to a factor of e.g., 1/10, 1/50, 1/100 or even more. As a further essential advantage, the detector device may be arranged for executing the time domain sampling of the amplified free induction decay light field synchronously with the optical parametric amplification, so that sections of the free induction decay light field are sampled in time domain in synchronism with the optical parametric amplification, i.e. each section of the free induction decay light field being sampled in time domain overlaps, preferably coincides, with the currently amplified section of the free induction decay light field. Thus, a time-resolved sensing of the free induction decay light field, e.g., like in [2], is provided, while the limitations of conventional techniques are avoided.

While the invention may be described by the excitation of the sample with the probe light pulse, the amplification of the free induction decay light field by the pump light pulse shorter than the free induction decay light field and the synchronous sampling of the amplified section of the free induction decay light field with the sampling light pulse shorter than the free induction decay light field, it is noted that the implementation of the invention is obtained with multiple pulses, preferably sequences of pulses, even at high repetition frequencies, e.g., up to the MHZ range, for successively amplifying different sections of the free induction decay light field with probe light pulses having different delay relative to the free induction decay light field.

Thus, for solving the above objective, the OPA pump light pulse is scanned across the free-induction decay synchronously with the EOS sampling light pulse, optionally using the same delay scanning stage. This will allow the OPA pump light pulse to be of femtosecond pulse duration that possesses a high peak power and at MHz repetition rate.

Advantageously, the rise time for amplification in OPA is on the femtosecond scale, compared to the QCL's tens of picosecond response time. This allows the amplification to be temporally close to but still separate from the excitation pulse, enabling the detection of fast-decaying signals.

According to a preferred embodiment of the invention, the amplifier device may include an amplifier delay stage being arranged for varying the amplification delay, and the detector device includes a sampling delay stage being arranged for varying the sampling delay. In terms of the method, the amplification delay may be varied with an amplifier delay stage of the amplifier device, and the sampling delay may be varied with a sampling delay stage of the detector device. Varying the amplification delay and the sampling delay means specifically adjusting the delays for each of the pump light pulses and sampling light pulses. Advantageously, the amplifier delay stage and the sampling delay stage facilitate a temporal adjustment of each pump pulse and each sampling light pulse, resp..

The amplifier delay stage and the sampling delay stage may be provided by separate delay stages or by a common main delay stage, as outlined below. Particularly preferred, the delay stages comprise beam path sections with varying beam path lengths being adjustable with mechanically moved reflectors. Preferably, the delay stage(s) may be configured for a periodic, particularly preferred continuous, change of the delay. Employing the common main delay stage provides the special advantage that synchronization between the OPA pump light pulse and EOS sampling light pulse is intrinsic, as they share the same delay scanning mechanism. No additional mechanisms, whether electronic or mechanical, are needed to track and control the relative delays between the EOS and OPA sampling and pump light pulses during scanning.

As a further advantage of the invention, various implementations of creating the probe, pump and sampling light pulses and synchronizing them are available. According to a first variant, the probe light source device, the pump light source device and the sampling light source device may be provided by separate synchronized pulse laser source devices being configured for an operation with a common repetition frequency. This variant has particular advantages in terms of specifically adjusting pulse parameters of the probe, pump and sampling light pulses. According to a second variant, the probe light source device, the pump light source device and the sampling light source device may be provided by a common main pulse laser source device. In this case, advantages in terms of reduced complexity of the setup of the spectroscopic measuring apparatus and/or facilitated control of the spectroscopic measuring method are obtained.

According to a further preferred embodiment of the invention, the amplifier delay stage and the sampling delay stage may be provided by a common main delay stage. In terms of the method, the amplification delay and sampling delay may be varied by the common main delay stage. Advantageously, the main delay stage may be commonly arranged in the beam paths of the pump light pulse and the sampling light pulse. Particularly preferred, the beam paths of the pump light pulse and the sampling light pulse coincide along at least one portion thereof. By setting the main delay stage, the amplification delay and the sampling delay are adjusted simultaneously, thus providing an inherent synchronism of the pump light pulse and the sampling light pulse and facilitating the control of the spectroscopic measurement.

According to an alternative advantageous embodiment of the invention, the spectroscopic measuring apparatus may be implemented without a physical delay stage for adjusting the temporal relationships of the probe, pump and sampling light pulses. With this embodiment, the probe light source device has a probe light repetition frequency and the pump light source has a pump light repetition frequency, being different from the probe light repetition frequency, and the sampling light source has a sampling light repetition frequency being equal to the pump light repetition frequency. Advantageously, a pulse-wise varying amplification delay is inherently provided by the detuning of the pump light repetition frequency relative to the probe light repetition frequency, and a pulse-wise varying sampling delay is inherently provided by the detuning of the sampling light repetition frequency relative to the probe light repetition frequency. Advantageously, this embodiment is inherently compatible with fast-scan techniques (heterodyne detection where the probe light pulse has a slightly different repetition rate compared to the OPA pump pulse and EOS detection pulse) and high-resolution dual-comb techniques.

Particularly preferred, the pump light source and the sampling light source may be provided by a common scanning pulse laser source device, thus allowing a reduction of the complexity of the optical setup in an advantageous manner.

As a further advantage, various techniques of time domain sampling can be employed for sensing the free induction decay light field. According to a preferred embodiment of the invention, the detector device may comprise an electro-optical sampling (EOS) detector device being arranged for an electro-optical sampling of the free induction decay light field with the sampling light pulses, wherein the OPA device and the EOS detector device are synchronized with each other. Advantageously, EOS detector devices are available from known FRS techniques. Alternatively, apart from EOS detector devices, synchronized OPA of the invention can also be applied to other time-domain techniques, such as time-domain techniques based on photo conductive switching or photo conductive antennas, as known from THz time domain spectroscopy, THz-induced lensing, infrared upconversion spectroscopy, or linear and nonlinear photoconductive sampling.

According to further preferred features of the invention, the probe pulse duration may be in a range from 3 fs to 500 fs, in particular from 3 fs to 300 fs, the pump pulse duration may be in a range from 30 fs to 300 fs, and the free induction decay duration may be in a range up to 10 ns, in particular 50 fs up to 100 ps, e.g., with solid or liquid samples, or up to 10 ns, e.g., with gaseous samples. For larger and more complex molecules, the free induction decay signal may decrease rapidly within 30 to 100 fs after the probe pulse has passed. Alternatively or additionally, a ratio of the free induction decay duration to the pump pulse duration may be selected in a range from 170 to 2. Advantageously, these parameter ranges are optimized for sensitive sensing of organic molecules, in particular molecules of biological samples. Furthermore, with these parameter ranges the free-induction-decay may be tracked as close to the probe pulse as possible, so that the strongest signal may be obtained but without being overwhelmed by the probe pulse itself.

According to a further optional embodiment of the invention, a pump intensity modulator may be provided for introducing a temporal modulation in the beam path of the pump light pulse. Advantageously, the pump intensity modulator allows to modulate (in particular: block/unblock) the pump light pulse beam path on a time scale longer than the duration of the free induction decay light field. The pump intensity modulator is arranged for transmitting the pump light pulse only during amplification of the free induction decay light field. This allows the amplification to occur when the delay is tuned such that the sample light pulse temporally overlaps with the free induction decay light field, and to shut down the amplification (by blocking the pump light pulse) when the delay is tuned such that the sample light pulse is temporally overlapping with the probe light pulse. Since the probe light pulse is much stronger than the free induction decay light field, by avoiding the amplification of the probe light pulse by the action of the pump intensity modulator, the dynamic range of detection can be adapted for measuring only the amplified free induction decay light field and the detector will not be saturated by an amplified probe light pulse.

Any device capable of modulating the pump intensity may be used, if modulation and optical properties are suitable, as the pump intensity modulator. With preferred examples, the pump intensity modulator may comprise e.g., a mechanical shutter or chopper (preferred in the case of slow scanning), or an acousto- or electro-optic modulator (preferred in the case of fast scanning).

Features disclosed in the context of the spectroscopic measuring apparatus and embodiments thereof also represent preferred features of the inventive spectroscopic measuring method and embodiments thereof. The aforementioned aspects and inventive and preferred features, in particular with regard to the configuration of the apparatuses as well as the dimensions and compositions of individual components being described in relation to the apparatuses, also apply for the methods. The preferred embodiments, variants and features of the invention described above are combinable with one another as desired.

### Brief description of the drawings

Further details and advantages of the invention are described in the following with reference to the attached drawings, which schematically show in:
- Figure 1:: features of a first embodiment of the invention, including a physical common main delay stage (amplifier and sampling delay stage);
- Figure 2:: the temporal relation of the probe light pulse, the sampling light pulse, the pump light pulse and the free induction decay light field;
- Figure 3:: features of a second embodiment of the invention, employing detuned repetition frequencies for providing the amplification delay and the sampling delay; and
- Figure 4:: a conventional FRS setup as disclosed in [2] (prior art).

### Preferred embodiments of the invention

Features of preferred embodiments of the invention are described in the following with reference to an FRS measuring apparatus and method employing an EOS detector device for field-resolved sensing of the free induction decay light field. Details of FRS, in particular details of creating the probe signal pulses, providing the sample and executing sensing with EOS, are not described as far as they are known per se from prior art, e.g., from [2]. The spectroscopic measuring apparatus may be provided with a control device (not shown in the Figures), like at least one computer unit, being coupled with at least one of the remaining components of the spectroscopic measuring apparatus and/or being arranged for collecting detector signals of the detector device and for calculating spectroscopic measuring data, in particular free induction decay light field data, of the sample to be investigated.

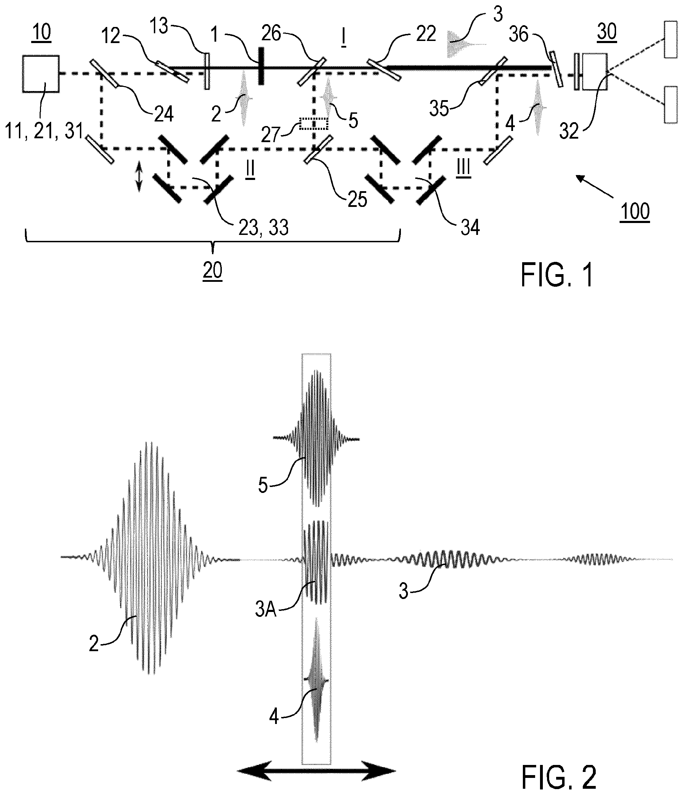

The invention is not restricted to the particular configuration of the spectroscopic measuring apparatus, but rather can be provided with a modified configuration, e. g. with regard to the detection principle. In particular, another time-domain technique may be employed instead of EOS. The application of the inventive method is not limited to the spectroscopic measuring apparatuses shown in Figures 1 or 3, but can be used in modified spectroscopy setups. Exemplary reference is made to a main pulse laser source device commonly representing a probe light source device, a pump light source device and a sampling light source device. Alternatively, separate probe light, pump light and/or sampling light source devices may be employed, wherein repetition rate and timing of the probe light, pump light and/or sampling light source devices are synchronized (synchronously controlled).

While the invention is described with reference to transmission mode measurements, wherein the light pulses are transmitted through the sample, the invention correspondingly may be applied with reflection mode measurements, wherein the light pulses are reflected at the sample.

Figure 1 illustrates a first embodiment of the inventive spectroscopic measuring apparatus 100 comprising a main pulse laser source device 10, an optical parametric amplification (OPA) device 20 and an electro-optic sampling (EOS) detector device 30 being arranged for measuring a spectral response of a sample 1. The sample 1 may be e.g., a liquid or vaporous biological sample.

The spectroscopic measuring apparatus 100 includes a first beam path I from the main pulse laser source device 10 via the sample 1 to the EOS detector device 30, a second beam path II from the main pulse laser source device 10 via a sampling delay stage 33 / amplifier delay stage 23 to an optically non-linear medium 22, and a third beam path III from the main pulse laser source device 10 via the sampling delay stage 33 / amplifier delay stage 23 to the EOS detector device 30.

The main pulse laser source device 10 commonly provides the laser oscillator source of a probe light source device 11 for creating probe light pulse 2 having a probe pulse duration, a pump light source device 21 for creating pump light pulse 5 having a pump pulse duration and a sampling light source device 31 for creating sampling light pulse 4 having a sampling pulse duration. The temporal relation of the probe light pulse 2, the sampling light pulse 4 and the pump light pulse 5 is illustrated in Figure 2 and further described below with reference to the inventive spectroscopic measuring method.

The laser oscillator source of the main pulse laser source device 10 comprises e.g., a Ho:YAG laser, Yb:YAG laser, Ti:Sa laser, Er-doped, Yb-doped or Ho-doped fiber laser, Cr:ZnS laser or Cr:ZnSe laser. Initially, the main pulse laser source device 10 creates a sequence of initial laser pulses with a repetition rate e.g., in a range from 100 kHz to 10 MHz or more (e.g., up to several hundreds of MHz), and with a center wavelength in the NIR and/or MIR wavelength range.

The probe light pulses 2 irradiating the sample 1 along the first beam path I are obtained by intra-pulse difference frequency mixing the initial laser pulses in an optically non-linear crystal 12. Residual light fields of the initial laser pulses are filtered out with a long pass filter 13 in the first beam path I. The probe pulse duration of the probe light pulses 2 is, e.g., fewer than 10 optical cycles (or shorter than 100 fs, in particular below 50 fs), and a probe light average power of the probe light pulses 2 is e.g., above 5 mW, preferably above 50 mW.

The OPA device 20 comprises the pump light source device 21 provided by the main pulse laser source device 10, the sampling delay stage 33 / amplifier delay stage 23, and the optically non-linear medium 22 arranged along the first beam path I. The pump light pulses 5 are provided by splitting a portion of the initial laser pulses at a first beam splitter 24 along the second/third beam paths II/III to the sampling delay stage 33 / amplifier delay stage 23 and by a further splitting at a second beam splitter 25 to the optically non-linear medium 22. With a first beam combiner 26, the pump light pulses 5 are superimposed with the free induction decay light field 3 for further propagation to the optically non-linear medium 22, either collinearly or non-collinearly.

Optionally, a pump intensity modulator 27 (schematically shown with dotted lines) can be provided in the second beam path II, e.g., in the beam path section between the second beam splitter 25 and the first beam combiner 26. The pump intensity modulator 27 comprises e.g., a fast scanning acousto- or electro-optic modulator which blocks the beam path such that, in the optically non-linear medium 22, no pump light pulse is superimposed with the light fields travelling along the first beam path during the duration of the probe light pulse. This may be obtained by controlling the pump intensity modulator 27 in synchronism with the probe light source device 11, e.g., by using the control device (not shown).

The schematically shown optically non-linear medium 22 comprises one or more OPA stages, which may be arranged in series, or in parallel (with output coherently recombined). Employing multiple OPA stages may have advantages in terms of spectrally broader phase-matching and broader optical bandwidth for amplification. The optically non-linear medium 22 in particular comprises one or more birefringent crystal(s) suitable for phase-matching the pump pulses and the free induction decay light fields (e.g. GaSe, ZGP), periodically poled crystals suitable for quasi-phase-matching (e.g. PPLN), or other nonlinear conversion elements that do not require uniform phase-matching structure such as polycrystalline materials that do not.

Anti-reflection coatings may be provided on the birefringent crystal(s) or periodically poled crystals for the specific wavelength to reduce reflection loss. The birefringent crystal(s) may have a thickness in a range from 50 µm to 5 mm, preferably from 300 µm to 3 mm.

A target gain factor of the free induction decay light field (including original excitation pulse) may range from ten to thousands or more, e.g., to several hundreds. The pump light pulses 5 may have a pump pulse duration from 30 fs to 300 fs and an average power from 1 to 20 Watts, preferably from 3 to 15 Watt, per OPA stage.

The sampling delay stage 33 / amplifier delay stage 23 may comprise one mechanical delay stage with electronic motion control (e.g. step-motor, servo-motor, piezo, galvanometric or acoustooptic scanner). The scanning range may be up to 5 ps with a scanning step size: in a range from 5 fs to 50 fs.

The detector device 30 comprises the sampling light source device 31 provided by the main pulse laser source device 10, the sampling delay stage 33 / amplifier delay stage 23, and the EOS detection system 32 arranged along the third beam path III. Additionally, the third beam path III may include an adjusting delay stage 34 being configured for initially setting up the electro-optic sampling. The sampling light pulses 4 with a sampling pulse duration of e.g., 3 fs to 30 fs, are provided by splitting a portion of the initial laser pulses at the first beam splitter 24 along the second/third beam paths II/III to the sampling delay stage 33 / amplifier delay stage 23 and by a further splitting at the second beam splitter 25 to the EOS detection system 32. With a second beam combiner 35, the sampling light pulses 4 are superimposed with the amplified free induction decay light fields 3, each including an amplified free induction decay light field section 3A, for further propagation via a short pass filter 36 to the EOS detection system 32. The EOS detection system 32 is configured as described e.g., in [2].

The spectroscopic measuring apparatus 100 is configured for measuring the spectral response of the sample 1 by a temporally resolved detection of the amplified free induction decay light field 3, as follows (see Figures 1 and 2).

The main pulse laser source device 10 creates a sequence of initial laser pulses which are used for providing a sequence of the probe pulses 2 irradiating the sample 1. With each probe pulse 2, the sample 1 is excited by an interaction of the light field of the probe pulse 2 with the sample 1. In response to the excitation, an immediate resonant oscillation light field (not shown in Figure 2) is created, followed by the free induction decay light field 3 of interest.

The free induction decay light field 3 is amplified by optical parametric amplification using a sequence of pump pulses 5. Because the pump pulses 5 have a pulse duration, e.g., 10 fs, below the duration of the free induction decay light field 3, e.g., 5 ps, each pump pulse amplifies a part of the free induction decay light field 3 only. By changing the delay between the free induction decay light field 3 and the pump pulse 5, a temporal scanning of the free induction decay light fields 3 with the pump pulses 5 is obtained (see double arrow in Figure 2). According to the current temporal position of a pump pulse 5 relative to the free induction decay light field 3, a certain section 3A of the free induction decay light field 3 (temporal overlap of the pump light pulse with the free induction decay light field) is amplified. With the changing delay set by the sampling delay stage 33 / amplifier delay stage 23, the pump pulses 3 are shifted relative to the free induction decay light field 3, so that the complete portion of interest of the free induction decay light field 3 is covered and amplified by successive pump pulses 5. Accordingly, a sequence of free induction decay light fields 3 is provided by the optically non-linear medium 22, wherein each of the free induction decay light fields 3 includes an amplified free induction decay light field section 3A at another temporal position of the free induction decay light field 3.

The detector device 30 is configured such that even the amplified free induction decay light field sections 3A of the free induction decay light fields 3 are sensed by electro-optical sampling. To this end, the sampling light pulses 4 with a sampling pulse duration equal to or shorter than the pump pulse duration are used for sampling the free induction decay light fields 3 each including the amplified free induction decay light field section 3A at another temporal position. In practice, the sampling pulse duration may be shorter than the pump pulse duration because it may be preferred if the sampling pulse is as short as possible, and that when the sampling pulse is shorter than the pump pulse, the amplified free induction decay light that the sampling pulse interacts with is less distorted by the increasing and reduction of amplification near the temporal edges of the pump pulse. The sampling light pulses 4 are synchronized with the pump pulses by the effect of the common creation with the main pulse laser source device 10 and the common delaying with the sampling delay stage 33 / amplifier delay stage 23. If there are path length differences of the second and third beam paths II, III, the adjusting delay stage 34 may be used for a compensation of such differences, so that the temporal position of each sampling pulse 4 is precisely tuned to the temporal position of one of the amplified free induction decay light field sections 3A of the free induction decay light fields 3. As a result of amplifying and synchronous sampling the free induction decay light fields 3 with the sequence of pump pulses 5 and sampling pulses 4, the compete free induction decay light field 3 is sensed and output as the spectral response of the sample 1 to be obtained.

Figure 3 illustrates the second embodiment of the inventive spectroscopic measuring apparatus 100 for measuring a spectral response of a sample 1, having a configuration with the main pulse laser source device 10, the optical parametric amplification (OPA) device 20 and the EOS detector device 30 like in Figure 1. Deviating from the first embodiment, the second embodiment does not include the sampling delay stage 33 / amplifier delay stage 23 of Figure 1, but employs detuned repetition frequencies of the pump and sampling light pulses 5, 4 relative to the probe light pulses 2 for providing the amplification and sampling delay.

As described above, the spectroscopic measuring apparatus 100 of Figure 3 includes the first beam path I from the main pulse laser source device 10 via the sample 1 to the EOS detector device 30, the second beam path II from the main pulse laser source device 10 via a repetition rate setting device 40 and via the sample 1 to an optically non-linear medium 22, and a third beam path III from the main pulse laser source device 10 via the repetition rate setting device 40 to the EOS detector device 30. Beam splitters, beam combiners and filters may be provided like in Figure 1. An adjusting delay stage 34 (not shown in Figure 3) may be provided for adjusting the EOS detection as described with reference to Figure 1.

With the embodiment of Figure 3, the main pulse laser source device 10 includes two different laser devices. A first one of the laser devices provides the probe light source device 11 for creating the probe light pulse 2, and another one of the laser devices commonly provides the pump light source device 21 for creating pump light pulse 5 and the sampling light source device 31. Both laser devices, i.e., the probe light source device 11 on the one side and the pump light source device 21 / sampling light source device 31 on the other side have different repetition frequencies, e.g., a difference in repetition frequency of 1 Hz for a probe light frequency of 25 MHz.

In operation, the detuned repetition frequency of the pump and sampling light pulses 5, 4 relative to the probe light pulses 2 inherently result in a pulse-wise changing delay of the pump light pulses 5 relative to the free induction decay light fields 3 at the optically non-linear medium 22 and a pulse-wise changing delay of the sampling light pulses 4 relative to the free induction decay light fields 3 at the input of the EOS detection system 32. Accordingly, a gated sensing of the amplified sections 3A (see Figure 2) of the free induction decay light fields 3 is obtained as described above.

The features of the invention disclosed in the above description, the drawings and the claims can be of significance both individually as well as in combination or sub-combination for the realization of the invention in its various embodiments. The invention is not restricted to the preferred embodiments described above. Rather a plurality of variants and derivatives is possible which also use the inventive concept and therefore fall within the scope of protection. In addition, the invention also claims protection for the subject and features of the sub claims independently of the features and claims to which they refer.

## Claims

1. Spectroscopic measuring apparatus (100) being configured for measuring a spectral response of a sample (1), in particular a biological sample, comprising:
- a probe light source device (11) being arranged for an irradiation of the sample (1) with a probe light pulse (2) having a probe pulse duration, wherein an interaction of the probe light pulse (2) with the sample (1) results in a free induction decay light field (3) having a free induction decay duration, which is greater than the probe pulse duration;
- an amplifier device being arranged for amplifying the free induction decay light field (3); and
- a detector device (30) being arranged for providing the spectral response of the sample by a temporally resolved detection of the free induction decay light field (3),
**characterized in that**
- the amplifier device is an optical parametric amplification (OPA) device (20) including a pump light source device (21) being arranged for repeatedly creating a pump light pulse (5) having a pump pulse duration, which is shorter than the free induction decay duration, and further including an optically non-linear medium (22), and
- the OPA device (20) is arranged for an optical parametric amplification of the free induction decay light field (3) by scanning the free induction decay light field (3) with the pump light pulse (5) repeatedly provided with a varying amplification delay relative to the free induction decay light field (3), and
- the detector device (30) includes a sampling light source device (31) being arranged for repeatedly creating a sampling light pulse (4) having a sampling pulse duration, which is shorter than the free induction decay duration, wherein the detector device (30) is arranged for time domain sampling of the free induction decay light field (3) by scanning the amplified free induction decay light field (3) with the sampling light pulse (4) repeatedly created with a varying sampling delay relative to the free induction decay light field (3), said sampling light pulse being provided synchronously with the pump light pulse (5).

2. Spectroscopic measuring apparatus according to claim 1, wherein
- the OPA device (20) includes an amplifier delay stage (23) being arranged for varying the amplification delay, and
- the detector device (30) includes a sampling delay stage (33) being arranged for varying the sampling delay.

3. Spectroscopic measuring apparatus according to claim 2, wherein
- the probe light source device (11), the pump light source device (21) and the sampling light source device (31) are provided by separate synchronized pulse laser source devices with a common repetition frequency.

4. Spectroscopic measuring apparatus according to claim 2, wherein
- the probe light source device (11), the pump light source device (21) and the sampling light source device (31) are provided by a common main pulse laser source device (10).

5. Spectroscopic measuring apparatus according to one of the claims 2 to 4, wherein
- the amplifier delay stage (23) and the sampling delay stage (33) are provided by a common main delay stage.

6. Spectroscopic measuring apparatus according to claim 1, wherein
- the probe light source device (11) has a probe light repetition frequency,
- the pump light source device (21) has a pump light repetition frequency being different from the probe light repetition frequency, and
- the sampling light source device (31) has a sampling light repetition frequency being equal to the pump light repetition frequency, wherein
- the varying amplification delay is provided by the detuning of the pump light repetition frequency relative to the probe light repetition frequency, and
- the varying sampling delay is provided by the detuning of the sampling light repetition frequency relative to the probe light repetition frequency.

7. Spectroscopic measuring apparatus according to claim 6, wherein
- the pump light source device (21) and the sampling light source device (31) are provided by a common scanning pulse laser source device.

8. Spectroscopic measuring apparatus according to one of the foregoing claims, wherein
- the detector device is an electro-optical sampling (EOS) detector device (30) being arranged for an electro-optical sampling of the free induction decay light fields (3) with the sampling light pulses, wherein
- the OPA device (20) and the EOS detector device (30) are synchronized with each other.

9. Spectroscopic measuring method for measuring a spectral response of a sample, in particular a biological sample, comprising the steps of:
- irradiation of the sample with a probe light pulse (2) created with a probe light source device (11) and having a probe pulse duration, wherein an interaction of the probe light pulse (2) with the sample results in a free induction decay light field (3) having a free induction decay duration, which is greater than the probe pulse duration;
- amplifying the free induction decay light field (3) with an amplifier device (20); and
- providing the spectral response of the sample by a temporally resolved detection of the free induction decay light field (3) with a detector device (30),
**characterized in that**
- the amplifier device is an optical parametric amplification (OPA) device (20) including a pump light source device (21) repeatedly creating a pump light pulse (5) having a pump pulse duration, which is shorter than the free induction decay duration, and further including an optically non-linear medium (22), and
- the OPA device (20) provides an optical parametric amplification of the free induction decay light field (3) by scanning the free induction decay light field (3) with the pump light pulse (5) repeatedly provided with a varying amplification delay relative to the free induction decay light field (3), and
- the detector device (30) includes a sampling light source device (31) repeatedly creating a sampling light pulse (4) having a sampling pulse duration, which is shorter than the free induction decay duration, wherein the detector device (30) provides a time domain sampling of the free induction decay light field (3) by scanning the amplified free induction decay light field (3) with the sampling light pulse (4) repeatedly created with a varying sampling delay relative to the free induction decay light field (3), said sampling light pulse (4) being synchronized with the pump light pulse (5).

10. Spectroscopic measuring method according to claim 9, wherein
- the amplification delay is varied with an amplifier delay stage (22) of the amplifier device (20), and
- the sampling delay is varied with a sampling delay stage (33) of the detector device (30).

11. Spectroscopic measuring method according to claim 10, wherein
- the probe light source, the pump light source and the sampling light source are provided by separate synchronized pulse laser source devices with a common repetition frequency, or
- the probe light source, the pump light source and the sampling light source are provided by a common main pulse laser source device.

12. Spectroscopic measuring method according to one of the claims 10 to 11, wherein
- the amplification delay and sampling delay are varied by a common main delay stage (23, 33).

13. Spectroscopic measuring method according to claim 9, wherein
- the probe light source has a probe light repetition frequency,
- the pump light source has a pump light repetition frequency being different from the probe light repetition frequency, and
- the sampling light source has a sampling light repetition frequency being equal to the pump light repetition frequency, wherein
- the varying amplification delay is provided by the detuning of the pump light repetition frequency relative to the probe light repetition frequency, and
- the varying sampling delay is provided by the detuning of the sampling light repetition frequency relative to the probe light repetition frequency.

14. Spectroscopic measuring method according to one of the claims 9 to 13, wherein
- the detector device (30) is an electro-optical sampling (EOS) detector device (30) being arranged for an electro-optical sampling of the free induction decay light fields (3) with the sampling light pulses (4), wherein
- the OPA device (20) and the EOS detector device (30) are synchronized with each other.

15. Spectroscopic measuring method according to one of the claims 9 to 14, wherein
- the probe pulse duration is in a range from 3 fs to 500 fs, in particular from 3 fs to 300 fs, the pump pulse duration is in a range from 30 fs to 300 fs, and/or the free induction decay duration is in a range up to 10 ps, in particular up to 5 ps, and/or
- a ratio of the free induction decay duration to the pump pulse duration is in a range from 170 to 2.
